# EUROPEAN PATENT APPLICATION

(11) **EP 4 481 052 A1**
(43) Date of publication of application: **25.12.2024**
(21) Application number: 23756449.7
(22) Date of filing: 16.02.2023
(51) Int. Cl.: C12P 19/26, C08B 37/00

(54) **COMPLEX-TYPE SUGAR ASPARAGINE AND METHOD FOR PRODUCING SAME**

(30) Priority: 18.02.2022 JP 2022024225
(71) Applicant: KH Neochem Co., Ltd., Tokyo 103-0022 (JP)
(72) Inventor: NAKAMACHI, Yuto, Kawasaki-shi, Kanagawa 212-0032 (JP)
(74) Representative: Mathys & Squire
(86) International application number: PCT/JP2023/005491
(87) International publication number: WO 2023/157924

(57) **Abstract**

The present invention relates to a complex-type glycoasparagine derived from an avian antibody, the complex-type glycoasparagine having a bisecting GlcNAc structure and a core fucose structure, wherein an Asn residue is optionally protected, and a method for producing the same.

## Description

### Technical Field

The present invention relates to a complex-type glycoasparagine and a method for producing the same.

### Background Art

Glycans are biopolymers in which monosaccharides are linked each other, and have a wide variety of structures because each monosaccharide as a basic binding unit has a plurality of binding points.

*In vivo* glycans are present in proteins or on cell surfaces and are involved in protein and cell recognition and signal transduction.

The glycans having such features are used in the medical field. For example, antibodies serving as active ingredients of antibody drugs usually have a glycan structure. Use of antibodies having homogeneous glycans is expected to lead to improvement in the quality and function of the antibody drugs, such as suppression of adverse reactions, improvement in drug efficacy, and extension of half-life in blood.

Abnormalities in glycan structures are known to be related to various diseases, and the utilization of glycans has been attempted to elucidate mechanisms of diseases. In addition to the elucidation of mechanisms of diseases, the application of glycans to diagnostic drugs has also been attempted.

Among the glycans having a wide variety of structures, glycans having a bisecting GlcNAc structure or a core fucose structure are known to be related to diseases such as cancers. Hence, attempts to extract glycans having a bisecting GlcNAc structure and/or a core fucose structure from living bodies to identify these structures, or to produce the structures by chemical synthesis have been made.

Non Patent Literature 1 discloses that the structure of a sugar moiety in chicken-derived serum IgG was analyzed. In Non Patent Literature 1, a compound derivatized through the reductive amination reaction of position 1 of reducing end N-acetylglucosamine with 2-aminopyridine (PA) was used for the structural analysis of the sugar moiety.

Non Patent Literatures 2 and 3 each disclose a chemical synthesis method for introducing a bisecting GlcNAc structure to a glycan.

Non Patent Literature 4 discloses a chemical synthesis method for introducing a bisecting GlcNAc structure and a core fucose structure to a glycan.

Non Patent Literature 5 discloses a synthesis method for introducing Gal to a bisecting GlcNAc-type glycan by enzymatic chemical synthesis.

### Citation List

### Non Patent Literature

Non Patent Literature 1: Glycobiology (2004) vol. 14, pp. 275-292
Non Patent Literature 2: Chem. Asian J. (2018) vol. 13, pp. 1544-1551
Non Patent Literature 3: Angew. Chem. Int. Ed. (2016) vol. 55, pp. 10487-10492
Non Patent Literature 4: Angew. Chem. Int. Ed. (2018) vol. 57, pp. 14543-14549
Non Patent Literature 5: ChemBioChem (2020) vol. 21, pp. 3212-3215

### Summary of Invention

### Technical Problem

However, Non Patent Literatures 1 to 5 disclose neither chemical synthesis of a complex-type glycoasparagine having a bisecting GlcNAc structure and a core fucose structure nor isolation of a complex-type glycoasparagine having a bisecting GlcNAc structure and a core fucose structure.

An object of the present application is to provide a novel complex-type glycoasparagine having a bisecting GlcNAc structure and a core fucose structure and a method for producing the same.

### Solution to Problem

The inventors of the present application have conducted diligent studies and consequently completed the present invention by finding a novel complex-type glycoasparagine having a bisecting GlcNAc structure and a core fucose structure and a method for producing the same.

Specifically, the present invention is as follows.
[1] A complex-type glycoasparagine derived from an avian antibody,
   the complex-type glycoasparagine having a bisecting GlcNAc structure and a core fucose structure, wherein an Asn residue is optionally protected.
[2] The complex-type glycoasparagine according to [1], wherein a glycan of the complex-type glycoasparagine has any of 9 to 13 monosaccharides, wherein the Asn residue is optionally protected.
[3] The complex-type glycoasparagine according to [1] or [2], wherein the complex-type glycoasparagine has the following structure: 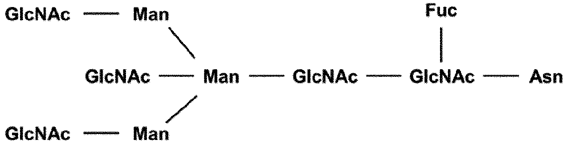 wherein the Asn residue is optionally protected.
[4] The complex-type glycoasparagine according to [3], wherein Gal or Neu5Ac-Gal is bound to GlcNAc in one or both of the GlcNAc-Man-Man moieties, wherein the Asn residue is optionally protected.
[5] A method for producing a complex-type glycoasparagine, comprising the steps of:
   providing a mixture containing a complex-type glycoasparagine derived from an avian antibody; and
   purifying the complex-type glycoasparagine by allowing the mixture to pass through an anion exchange resin,
   wherein in the purification step, the complex-type glycoasparagine is separated on the basis of the number of sialic acid residues.
[6] The method according to [5], wherein the mixture provision step comprises the steps of:
   denaturing the avian antibody; and
   degrading the denatured avian antibody with a proteolytic enzyme.
[7] The method according to [6], wherein in the avian antibody denaturation step, the avian antibody is denatured with heat or an organic solvent.

### Advantageous Effects of Invention

The present invention can provide a novel complex-type glycoasparagine having a bisecting GlcNAc structure and a core fucose structure and a method for producing the same.

### Brief Description of Drawings

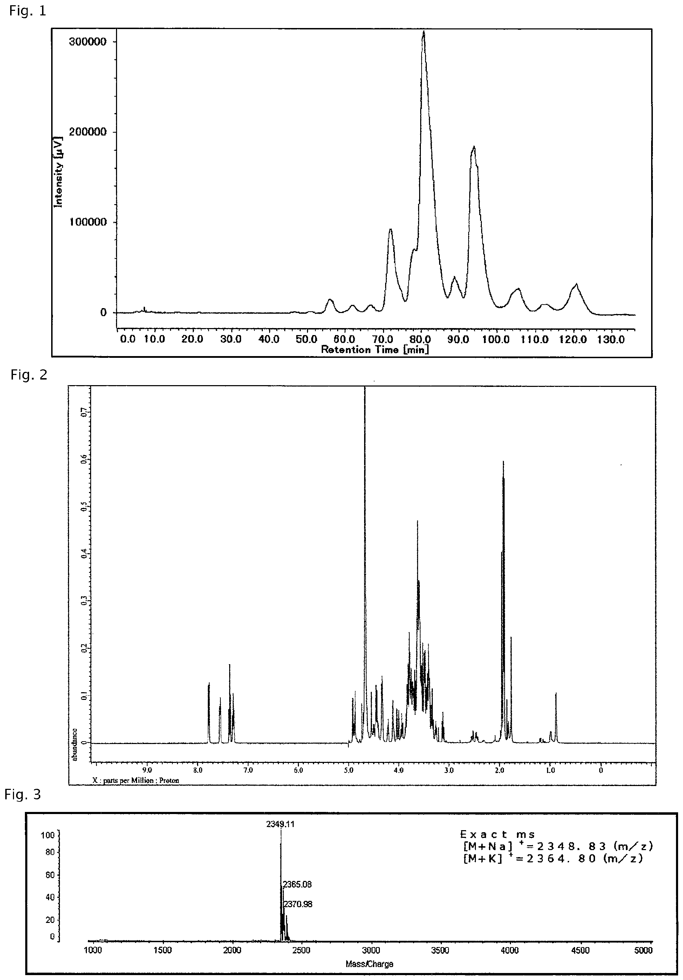

[Figure 1] Figure 1 shows results of reversed-phase chromatography involving separation on the basis of neutral glycoasparagine derivatives differing in the number of monosaccharides in Example 2.
[Figure 2] Figure 2 shows ¹H-NMR of a complex-type glycoasparagine derivative of 11 monosaccharides having 0 sialic acid residue and having a bisecting GlcNAc structure and a core fucose structure, which was obtained from a fraction eluted approximately 80 minutes later in reversed-phase chromatography.
[Figure 3] Figure 3 shows mass spectrometry results of a complex-type glycoasparagine derivative of 11 monosaccharides having 0 sialic acid residue and having a bisecting GlcNAc structure and a core fucose structure, which was obtained from a fraction eluted approximately 80 minutes later in reversed-phase chromatography.

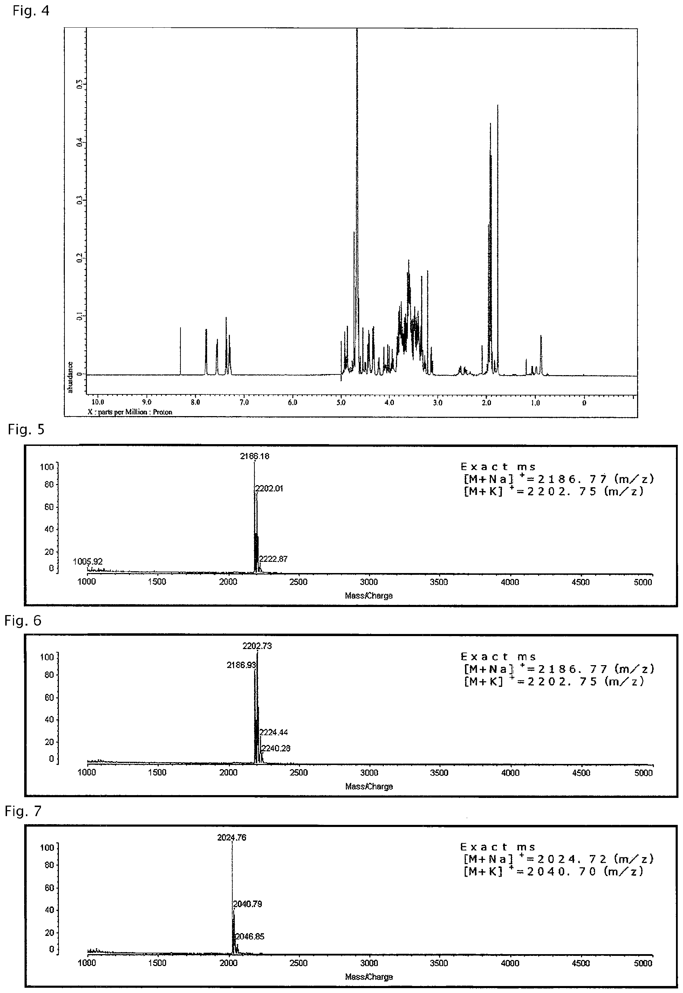

[Figure 4] Figure 4 shows ¹H-NMR of a complex-type glycoasparagine derivative of 10 monosaccharides having 0 sialic acid residue and having a bisecting GlcNAc structure and a core fucose structure, which was obtained from a fraction eluted approximately 92 minutes later in reversed-phase chromatography.
[Figure 5] Figure 5 shows mass spectrometry results of a complex-type glycoasparagine derivative of 10 monosaccharides having 0 sialic acid residue and having a bisecting GlcNAc structure and a core fucose structure, which was obtained from a fraction eluted approximately 92 minutes later in reversed-phase chromatography.
[Figure 6] Figure 6 shows mass spectrometry results of a complex-type glycoasparagine derivative of 10 monosaccharides having 0 sialic acid residue and having a bisecting GlcNAc structure and a core fucose structure, which was obtained from a fraction eluted approximately 102 minutes later in reversed-phase chromatography.
[Figure 7] Figure 7 shows mass spectrometry results of a complex-type glycoasparagine derivative of 9 monosaccharides having 0 sialic acid residue and having a bisecting GlcNAc structure and a core fucose structure, which was obtained from a fraction eluted approximately 118 minutes later in reversed-phase chromatography.

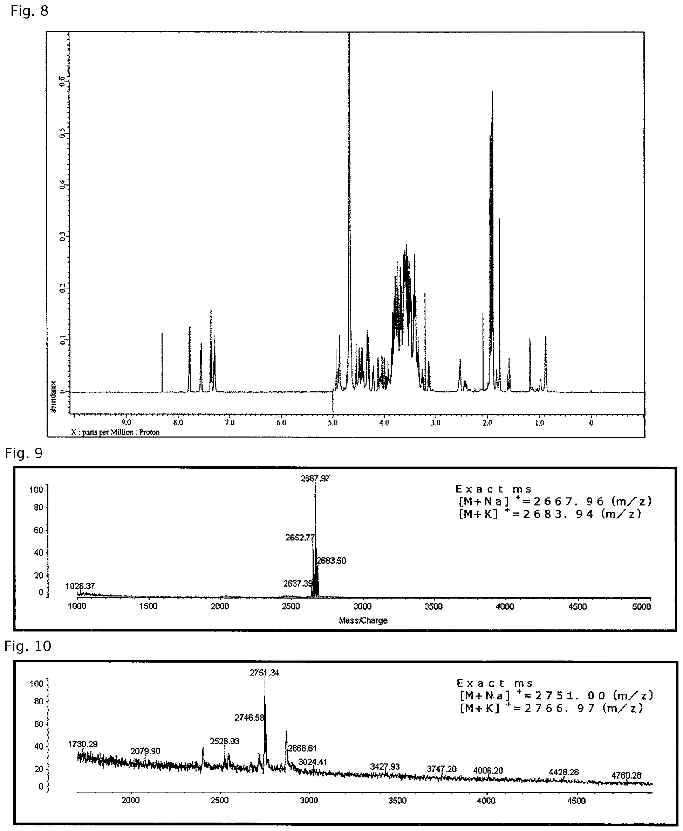

[Figure 8] Figure 8 shows ¹H-NMR of a complex-type glycoasparagine derivative of 12 monosaccharides having one sialic acid residue and having a bisecting GlcNAc structure and a core fucose structure.
[Figure 9] Figure 9 shows mass spectrometry results of a complex-type glycoasparagine derivative of 12 monosaccharides having one sialic acid residue and having a bisecting GlcNAc structure and a core fucose structure.
[Figure 10] Figure 10 shows mass spectrometry results of a complex-type glycoasparagine derivative of 13 monosaccharides having two sialic acid residues and having a bisecting GlcNAc structure and a core fucose structure.

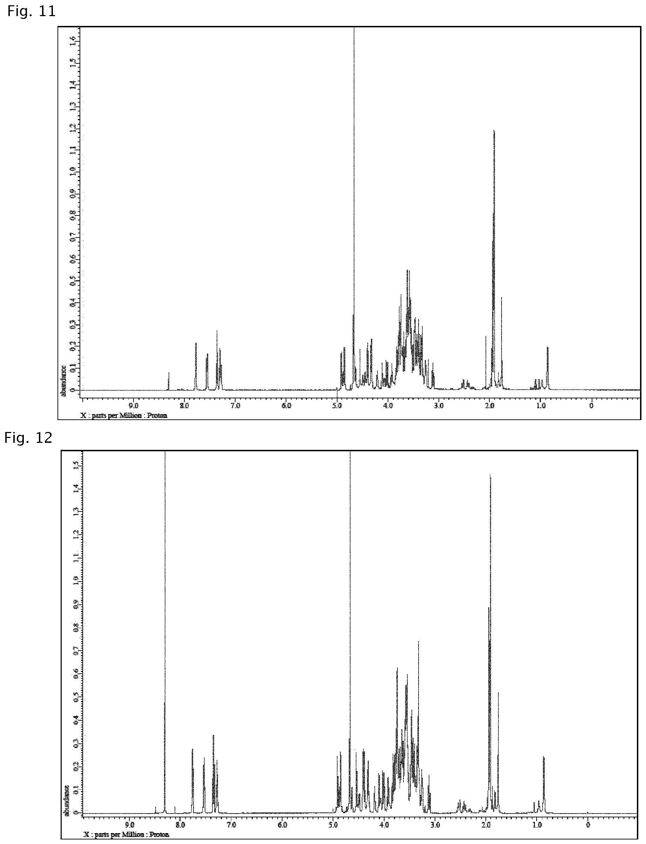

[Figure 11] Figure 11 shows ¹H-NMR of a complex-type glycoasparagine derivative of 10 monosaccharides having 0 sialic acid residue and having a bisecting GlcNAc structure and a core fucose structure, which was obtained from a fraction eluted approximately 102 minutes later in reversed-phase chromatography.
[Figure 12] Figure 12 shows ¹H-NMR of a complex-type glycoasparagine derivative of 9 monosaccharides having 0 sialic acid residue and having a bisecting GlcNAc structure and a core fucose structure, which was obtained from a fraction eluted approximately 118 minutes later in reversed-phase chromatography.

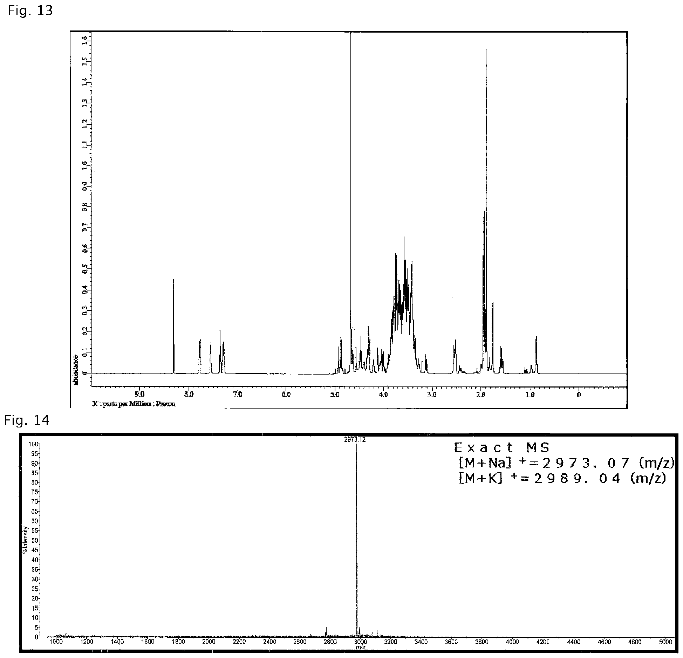

[Figure 13] Figure 13 shows ¹H-NMR of a complex-type glycoasparagine derivative of 13 monosaccharides having two sialic acid residues and having a bisecting GlcNAc structure and a core fucose structure.
[Figure 14] Figure 14 shows mass spectrometry results of a complex-type glycoasparagine derivative of 13 monosaccharides having two sialic acid residues and having a bisecting GlcNAc structure and a core fucose structure.

### Description of Embodiments

Hereinafter, the mode for carrying out the present invention will be described in detail. The present invention is not limited by embodiments given below and can be carried out by making various changes or modifications without departing from the spirit of the present invention.

### (Complex-type glycoasparagine)

The complex-type glycoasparagine of the present invention is a complex-type glycoasparagine derived from an avian antibody and has a bisecting GlcNAc structure and a core fucose structure.

The complex-type glycoasparagine has a basic skeleton in which complex-type sugars are bound to asparagine. In general, glycans of high mannose, hybrid, and complex types are known as N-linked glycans. The complex-type glycoasparagine of the present invention has a complex-type glycan among the N-linked glycans.

The complex-type glycoasparagine of the present invention is a complex-type glycoasparagine derived from an avian antibody and has a complex-type glycoasparagine structure contained in the avian antibody. Thus, the whole or a portion of the structure of the complex-type glycoasparagine of the present invention may be identical to the complex-type glycoasparagine structure contained in the avian antibody.

In this context, the avian antibody is an antibody that can be obtained from a bird. While the avian antibody is not particularly limited, the whole amino acid sequence of the antibody is derived from a bird.

Examples of the bird include chickens, gooses, and ducks. A chicken is preferred.

Examples of the isoform of the avian antibody include, but are not particularly limited to, IgY, IgA, and IgM. IgY is preferred.

The avian antibody can be obtained from an egg, blood, or the like of a bird by a conventionally known method. While the avian antibody is not particularly limited, an antibody derived from an egg may be used. In particular, the avian antibody can be an antibody derived from an egg of a chicken, a goose, a duck, or the like. An antibody derived from a chicken egg may be used because a large amount of IgY can be obtained from the chicken egg.

The avian antibody will be described with reference to IgY. IgY has a Fab region and a Fc region.

The Fab region of the avian antibody is composed of a heavy chain and a light chain linked to each other through a disulfide bond, and linked to the Fc region via a hinge region.

The complex-type glycoasparagine derived from an avian antibody according to the present invention is a complex-type glycoasparagine contained in the avian antibody and therefore, can be a complex-type glycoasparagine derived from IgY, IgA, or IgM and can be a complex-type glycoasparagine derived from an antibody fragment such as an IgY Fab region or Fc region.

The complex-type glycan preferably has a structure called core pentasaccharide bound to asparagine via a reducing end.

The structure of the core pentasaccharide bound to asparagine via a reducing end includes the following structure:

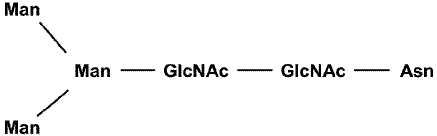

In the present specification, Asn represents asparagine, Man represents mannose, GlcNAc represents N-acetylglucosamine, Neu5Ac represents sialic acid, Gal represents galactose, and Fuc represents fucose. In the structure described above, two Man residues positioned at the left end form a non-reducing end.

The straight lines between the individual sugars and between GlcNAc and Asn each represent a bond. Each bond is not particularly limited as long as the bond is present in a structure derived from the avian antibody. An α bond or a β bond can be used.

The glycan structure in the complex-type glycoasparagine of the present invention may be a structure bound to N-acetylglucosamine, sialic acid, galactose, fucose, or the like, in addition to the structure of the core pentasaccharide. N-Acetylglucosamine, sialic acid, galactose, or the like may be bound on the non-reducing end side of the core pentasaccharide. The complex-type glycoasparagine of the present invention may further have a branched structure as a structure on the non-reducing end side.

### (Bisecting GlcNAc structure)

The bisecting GlcNAc structure is a structure having N-acetylglucosamine bonded to mannose bonded to GlcNAc in the core pentasaccharide of an N-linked glycan.

### (Core fucose structure)

The core fucose structure is a structure having fucose bonded to reducing end N-acetylglucosamine bonded to Asn in the core pentasaccharide of an N-linked glycan.

The glycan having the bisecting GlcNAc structure and the core fucose structure preferably has the following structure:

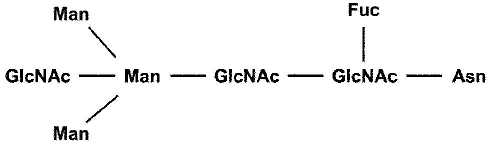

In the structure described above, GlcNAc bonded to Man described as a non-reducing end corresponds to the bisecting GlcNAc structure, and Fuc corresponds to the core fucose structure.

While the glycan structure of the complex-type glycoasparagine is not particularly limited, a complex-type glycoasparagine having a glycan of any of 9 to 13 monosaccharides is preferred.

The glycan of 9 monosaccharides has, for example, the following structure:

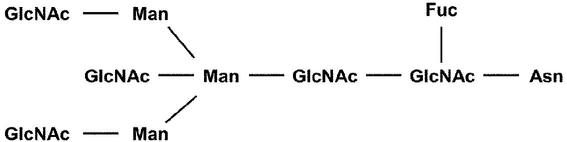

In the glycan structures of 10 to 13 monosaccharides, galactose or sialic acid may be bound to GlcNAc in one or both of the GlcNAc-Man- moieties in the glycan of 9 monosaccharides. In this case, galactose or sialic acid is preferably bound to one or both of the GlcNAc residues which are not GlcNAc of the bisecting GlcNAc structure.

Each bond between the sugars is not particularly limited as long as the position of a hydroxy group of the sugar on the reducing end side to which the sugar on the non-reducing end side is bound is also a position present in a structure derived from the avian antibody. Each bond between the sugars can be a 1->2 bond, a 1-->3 bond, a 1-->4 bond, or a 1-->6 bond. Each of the 1->2 bond, the 1-->3 bond, the 1-->4 bond, and the 1-->6 bond may be an α bond or may be a β bond.

The sugar-asparagine bond is usually a 1-->N bond, preferably a β1→N bond.

Each of the N-acetylglucosamine, the mannose, the galactose, and the sialic acid is usually a D-sugar.

The fucose is usually an L-sugar.

When the glycan structure of the complex-type glycoasparagine is a glycan structure of any of 9 to 13 monosaccharides, a glycan structure having a structure specifically described below is preferred.

The glycan moiety of the complex-type glycoasparagine, when having 9 monosaccharides, can have a structure given below.

In the structure given below, for example, β1→2 in the bond between GlcNAc and Man means that N-acetylglucosamine on the non-reducing end side is bound at position 1 through a β bond to a hydroxy group at position 2 of mannose on the reducing end side. β1→N means that N-acetylglucosamine on the reducing end side is bound at position 1 through a β bond to N of Asn. The same holds true for glycans of 9 to 13 monosaccharides given below.

### Man3GlcNAc5Fuc (9 monosaccharides)

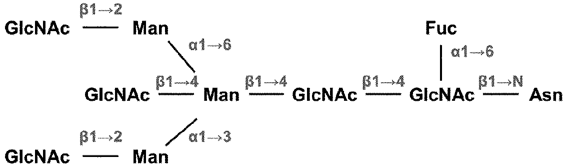

The glycan moiety of the complex-type glycoasparagine, when having 10 monosaccharides, can have the following structure:

### GalMan3GlcNAc5Fuc (10 monosaccharides) (1)

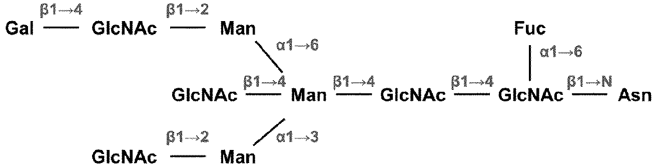

### GalMan3GlcNAc5Fuc (10 monosaccharides) (2)

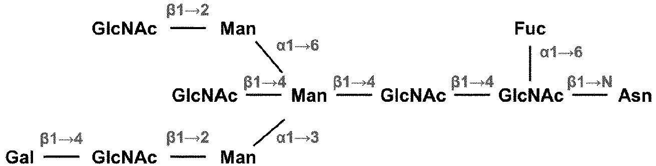

The glycan moiety of the complex-type glycoasparagine, when having 11 monosaccharides can have the following structure:

### Gal2Man3GlcNAc5Fuc (11 monosaccharides) (1)

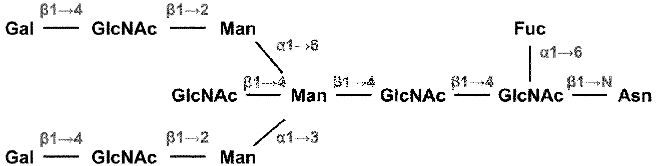

### Neu5AcGalMan3GlcNAc5Fuc (11 monosaccharides) (2)

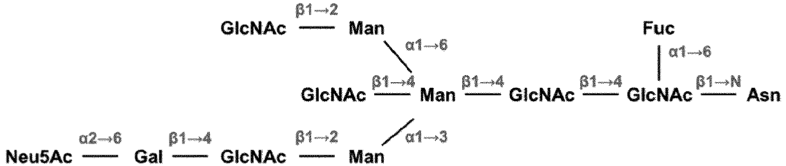

### Neu5AcGalMan3GlcNAc5Fuc (11 monosaccharides) (3)

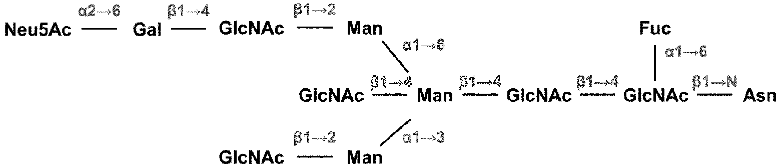

The glycan moiety of the complex-type glycoasparagine, when having 12 monosaccharides, can have the following structure:

### Neu5AcGal2Man3GlcNAc5Fuc (12 monosaccharides) (1)

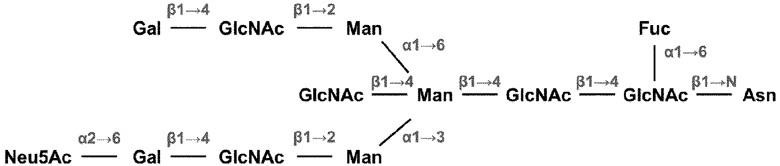

### Neu5AcGal2Man3GlcNAc5Fuc (12 monosaccharides) (2)

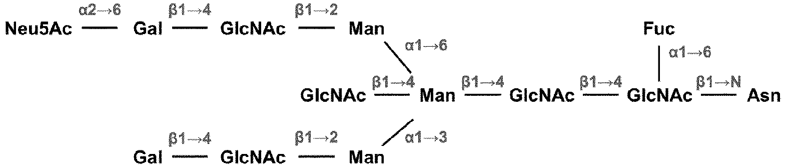

The glycan moiety of the complex-type glycoasparagine, when having 13 monosaccharides, can have the following structure:

### Neu5Ac2Gal2Man3GlcNAc5Fuc (13 monosaccharides)

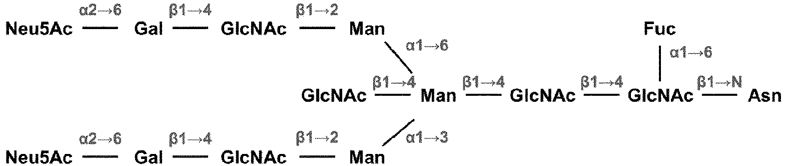

The asparagine in the form of L-asparagine has a structure given below. The asparagine in the N-linked glycan resides at the N terminus, and the asparagine is bound through a CONH₂ group to the glycan.

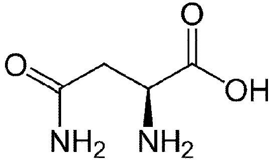

The asparagine in the complex-type glycoasparagine of the present invention may contain an NH₂ group and a COOH group in a free state, as a moiety except for the CONH₂ group bound to the glycan. Therefore, the amino group and the carboxyl group are optionally protected.

The optionally protected Asn residue means that an amino group and/or a carboxyl group in the asparagine residue is/are protected.

When the asparagine residue is protected, the term "protected" means being protected with a conventionally known protective group for an amino acid, specifically, a protective group for an amino group or a carboxyl group.

The conventionally known protective group for an amino acid that may be used as a protective group for an amino group or a carboxyl group can be selected from, for example, protective groups described in Greene's Protective Groups in Organic Synthesis.

Examples of the protective group for an amino group include, but are not particularly limited to, a Fmoc group, a t-butoxycarbonyl (Boc) group, a benzyloxycarbonyl (Cbz or Z) group, a p-methoxybenzyloxycarbonyl (Z(OMe) or pMZ) group, and a 2-(p-biphenyl)isopropyloxycarbonyl (Bpoc) group.

Examples of the protective group for a carboxyl group include, but are not particularly limited to, a methyl ester group, an ethyl ester group, a benzyl ester group, and a t-butyl ester group. Each of the methyl ester group, the ethyl ester group, the benzyl ester group, and the t-butyl ester group is a protective group of ester formed by methyl alcohol, ethyl alcohol, benzyl alcohol, and t-butyl alcohol, respectively, with a carboxyl group.

The amino group or the carboxyl group of the asparagine may be present in a free state (NH₂ or COOH), may be present as NH₃⁺ or COO⁻, or may be a combination thereof.

The amino group or the carboxyl group of the asparagine may be in a salt state. In this context, the salt is not particularly limited and may be, for example, an inorganic acid salt such as hydrochloride, sulfate, or phosphate, or an organic acid salt such as acetate, propionate, tartrate, fumarate, maleate, malate, citrate, methanesulfonate, p-toluenesulfonate, or trifluoroacetate. The salt may be, for example, an alkali metal salt or an alkaline earth metal salt, such as sodium salt, potassium salt, calcium salt, or magnesium salt.

### (Method for producing complex-type glycoasparagine)

The method for producing a complex-type glycoasparagine according to the present invention is a method for producing a complex-type glycoasparagine, comprising the steps of:
providing a mixture containing a complex-type glycoasparagine derived from an avian antibody; and
purifying the complex-type glycoasparagine by allowing the mixture to pass through an anion exchange resin,
wherein in the purification step, the complex-type glycoasparagine is separated on the basis of the number of sialic acid residues.

### (Provision of mixture containing complex-type glycoasparagine)

In the step of providing a mixture containing a complex-type glycoasparagine derived from an avian antibody, the mixture to be provided contains a complex-type glycoasparagine cleaved from an avian antibody.

The complex-type glycoasparagine cleaved from an avian antibody usually comprises plural types of complex-type glycoasparagine units.

The mixture is not particularly limited as long as the mixture contains one or more types of complex-type glycoasparagine units. The mixture may contain an avian antibody-derived component other than the complex-type glycoasparagine according to the present invention.

The avian antibody-derived component other than the complex-type glycoasparagine may be glycoasparagine other than the complex-type glycoasparagine of the present invention or may be a peptide fragment, a glycan, an amino acid, or a glycopeptide derived from the avian antibody.

The mixture may also contain a component that is not derived from the avian antibody. Examples of the component that is not derived from the avian antibody include, but are not particularly limited to, components necessary for denaturing or degrading the avian antibody.

Any avian antibody can be applied to the avian antibody for use in the method for producing a complex-type glycoasparagine with reference to the description about the avian antibody described in relation to the complex-type glycoasparagine, and an avian antibody obtained by a conventionally known method can be used.

The avian antibody is not particularly limited and can be an antibody derived from a bird, and the whole amino acid sequence of the antibody is derived from a bird.

Examples of the bird include chickens, gooses, and ducks. A chicken is preferred.

Examples of the isoform of the avian antibody include, but are not particularly limited to, IgY, IgA, and IgM. IgY is preferred.

The avian antibody can be obtained from an egg, blood, or the like of a bird by a conventionally known method. While the avian antibody is not particularly limited, an antibody derived from an egg may be used. In particular, the avian antibody can be an antibody derived from an egg of a chicken, a goose, a duck, or the like, and an antibody derived from a chicken egg may be used.

IgY, IgA, IgM, or the like may be used directly as the avian antibody, or a fraction containing an antibody fragment such as a Fab region and/or a Fc region obtained by a conventionally known method may be used.

In the case of using an antibody fragment such as an avian antibody Fab region, F(ab')₂ region, or Fc region in providing a mixture containing a complex-type glycoasparagine derived from an avian antibody, the method further comprises a step of treating the avian antibody with a proteolytic enzyme capable of fragmenting an antibody, for example, papain or pepsin.

The antibody fragment such as the avian antibody Fab region, F(ab')₂ region, or Fc region obtained by fragmenting the avian antibody may be used for providing a mixture containing a complex-type glycoasparagine derived from an avian antibody.

A component other than the antibody fragment such as the Fab region, the F(ab')₂ region, or the Fc region obtained by fragmenting the avian antibody may be removed by purification by a conventionally known method.

### (Degradation of avian antibody)

The step of providing a mixture containing a complex-type glycoasparagine derived from an avian antibody may comprise the step of degrading the avian antibody. The avian antibody in the avian antibody degradation step means IgY, IgA, IgM, or the like, or an antibody fragment thereof.

Examples of the antibody fragment include, but are not particularly limited to, Fab regions, F(ab')₂ regions, and Fc regions.

The method for degrading the avian antibody is not particularly limited as long as the avian antibody can be degraded to obtain a mixture containing a complex-type glycoasparagine. The avian antibody can be degraded by a conventionally known method.

For the degradation of the avian antibody, the avian antibody may be degraded with, for example, a proteolytic enzyme.

By the degradation of the avian antibody with the proteolytic enzyme, complex-type sugars are cleaved, together with asparagine bound thereto in an amino acid sequence constituting the avian antibody, as a complex-type glycoasparagine from the avian antibody.

The reaction solution of the avian antibody with the proteolytic enzyme consequently contains a mixture containing a complex-type glycoasparagine derived from the avian antibody.

The reaction solution of the avian antibody with the proteolytic enzyme may also contain an avian antibody-derived component other than the complex-type glycoasparagine.

The proteolytic enzyme for use in the method for degrading the avian antibody is not particularly limited as long as the proteolytic enzyme is a degrading enzyme capable of cleaving asparagine to which complex-type sugars are bound. Examples thereof include actinase E, pronase, and Orientase.

In particular, actinase E is preferred from the viewpoint of substrate specificity.

The reaction temperature with the proteolytic enzyme is not particularly limited as long as the proteolytic enzyme retains enzymatic activity. The temperature is, for example, in the range of 25°C to 50°C, preferably in the range of 35°C to 45°C.

The reaction time with the proteolytic enzyme is not particularly limited as long as the mixture containing the complex-type glycoasparagine can be obtained. The time is, for example, in the range of 12 hours to 168 hours, preferably in the range of 24 hours to 96 hours.

After reaction with the proteolytic enzyme, for example, a reaction solution obtained by inactivating the proteolytic enzyme may be used as the mixture containing the complex-type glycoasparagine. The reaction solution thus obtained by the deactivation of the proteolytic enzyme may be further partially purified by column chromatography or the like, and the resulting solution can be used as the mixture containing the complex-type glycoasparagine.

The inactivation of the proteolytic enzyme or the partial purification by column chromatography or the like can be carried out by a conventionally known method.

In particular, the step of providing a mixture containing a complex-type glycoasparagine derived from an avian antibody preferably further comprises a step of removing an amino acid, a peptide, and the like contained in the mixture containing the complex-type glycoasparagine.

Examples of a conventionally known step of removing an amino acid, a peptide, and the like that may be used include purification methods using a cotton column. The amino acid, the peptide, and the like may be removed by a method described in, for example, Chinese J. Anal. Chem., (2020), vol. 48 (1), pp. 34-39, as a purification method using a cotton column.

### (Denaturation of avian antibody)

The step of providing a mixture containing a complex-type glycoasparagine may further comprise a step of denaturing the avian antibody as a step previous to the step of degrading the avian antibody.

The method for denaturing the avian antibody is not particularly limited as long as the conformation of the avian antibody can be altered. The avian antibody can be denatured by a conventionally known method.

For example, a denaturation method with heat or an organic solvent can be used for denaturing the avian antibody.

Before degradation of the avian antibody, the conformation of the avian antibody can be altered by denaturing the avian antibody. The reactivity of degradation of the avian antibody with the proteolytic enzyme can thereby be improved.

In the case of denaturing the avian antibody by heat treatment, the heat treatment is preferably carried out under reaction conditions as described below from the viewpoint of reactivity with the proteolytic enzyme.

The heat treatment can be performed by directly applying heat to a solution containing the avian antibody for a given time.

The reaction temperature is not particularly limited. The temperature is, for example, in the range of 65°C to 95°C, preferably in the range of 70°C to 90°C, more preferably in the range of 70°C to 80°C.

The reaction time is not particularly limited. The time is, for example, in the range of 1 minute to 20 minutes, preferably in the range of 5 minutes to 10 minutes.

The solvent for the solution containing the avian antibody is not particularly limited and is, for example, preferably water and may be a buffer solution or an aqueous solution.

In the case of denaturing the avian antibody by organic solvent treatment, the organic solvent treatment is preferably carried out under reaction conditions as described below from the viewpoint of reactivity with the proteolytic enzyme.

The organic solvent treatment can be performed by adding an organic solvent to a solution containing the avian antibody, and retaining the mixture for a given time.

Examples of the organic solvent include, but are not particularly limited to, acetone, methanol, ethanol, 2-propanol, chloroform, and ethyl acetate.

One solvent may be used as the organic solvent, or a mixed solvent of two or more solvents may be used.

As for a water-soluble organic solvent, the avian antibody may be denatured with an aqueous solution containing the organic solvent.

The reaction time is not particularly limited. The time is, for example, in the range of 1 hour to 24 hours, preferably in the range of 3 hours to 12 hours.

The reaction temperature is not particularly limited and can be, for example, room temperature.

In the organic solvent treatment, the avian antibody may be directly immersed in or brought into contact with the organic solvent. Alternatively, the organic solvent may be added to a solution containing the avian antibody, or a solution containing the avian antibody may be added to the organic solvent.

### (Purification of complex-type glycoasparagine)

The step of purifying the complex-type glycoasparagine is the purification step of allowing the mixture containing the complex-type glycoasparagine derived from an avian antibody, which has been obtained by the previous step, to pass through an anion exchange resin.

The mixture before being subjected to this step contains a complex-type glycoasparagine cleaved from the avian antibody and usually contains plural types of complex-type glycoasparagine units. By this step, the complex-type glycoasparagine contained in the mixture is separated on the basis of the number of sialic acid residues.

The number of sialic acid residues contained in the complex-type glycoasparagine is not particularly limited and is, for example, 0 to 2.

The phrase "complex-type glycoasparagine contained in the mixture is separated on the basis of the number of sialic acid residues" means that complex-type glycoasparagine units can be sequentially eluted according to the number of sialic acid residues into eluates from the anion exchange resin, and thereby separated and fractionated as respective fractions of a complex-type glycoasparagine having, for example, 0, 1, or 2 sialic acid residues.

Each of the fraction of the complex-type glycoasparagine having 0 sialic acid residues, the fraction of the complex-type glycoasparagine having one sialic acid residue, and the fraction of the complex-type glycoasparagine having two sialic acid residues can contain plural types of complex-type glycoasparagine units.

Before purifying the complex-type glycoasparagine by allowing the mixture containing the complex-type asparagine to pass through an anion exchange resin, the mixture containing the complex-type asparagine may be partially purified by a conventionally known method.

Examples of the anion exchange resin include, but are not particularly limited to, strong anion exchange resins and weak anion exchange resins.

Examples of the base material of the anion exchange resin include, but are not particularly limited to, styrene, divinylbenzene, cellulose, and agarose.

Examples of the strong anion exchange resin include a base material in which a quaternary amino group is introduced.

Examples of the weak anion exchange resin include a base material in which a tertiary amino group, a diethylaminoethyl group, a carboxylmethyl group, polyamine, or the like is introduced.

Examples of the purification method for allowing the mixture containing the complex-type glycoasparagine to pass through the anion exchange resin include, but are not particularly limited to, anion exchange chromatography.

A conventionally known buffer solution can be used as a mobile phase for use in anion exchange chromatography.

Examples of the solvent for use in the buffer solution include, but are not particularly limited to, water, acetonitrile, and alcohols. One solvent may be used, or a mixed solvent of two or more solvents may be used.

Examples of the component of the buffer solution include, but are not particularly limited to, components that retain buffering ability, such as acetate, phosphate, citrate, alkylamine, ammonium, trishydroxymethylaminomethane, and pyridine. One component may be used, or two or more components may be used.

The pH of the mobile phase is not particularly limited and is preferably in the range of neutral to basic pH, more preferably in the range of pH 7 to pH 10.

In the step of purifying the mixture containing the complex-type glycoasparagine, elution may be performed with a mobile phase under gradient conditions by anion exchange chromatography. In the case of using, for example, a mobile phase under gradient conditions as the mobile phase, elution may be performed under the gradient conditions in the pH range of 7 to 10.

The method for producing a complex-type glycoasparagine according to the present invention may comprise, for further purification, a step of purifying the complex-type glycoasparagine separated on the basis of the number of sialic acid residues. In the step of purifying the complex-type glycoasparagine separated on the basis of the number of sialic acid residues, the complex-type glycoasparagine separated on the basis of the number of sialic acid residues may be further purified in order to enhance purity.

The complex-type glycoasparagine separated on the basis of the number of sialic acid residues may comprise glycoasparagine units differing in glycan structure though having the same number of sialic acid residues. Therefore, the glycoasparagine units differing in glycan structure may be further purified.

The method for further purification is not particularly limited, and a conventionally known method for purifying a glycopeptide such as glycoasparagine can be used.

In particular, the method for further purification may involve introducing a protective group to asparagine in order to perform separation and purification, though the complex-type glycoasparagine separated on the basis of the number of sialic acid residues by anion exchange chromatography may be used directly in the subsequent purification step.

The glycoasparagine units differing in glycan structure may be separated and purified by introducing a protective group to the asparagine of the complex-type glycoasparagine. In particular, the glycoasparagine units differing in glycan structure may be separated and purified by introducing a lipid-soluble protective group thereto.

The method for producing a complex-type glycoasparagine according to the present invention may comprise, for further purification, a step of introducing a protective group to the complex-type glycoasparagine separated on the basis of the number of sialic acid residues.

The reaction of introduction of the protective group to the complex-type glycoasparagine is not particularly limited and can be carried out by a conventionally known method.

Any protective group can be applied to the protective group to be introduced to the glycoasparagine with reference to the description in relation to the protective group for the asparagine. In particular, examples thereof include protective groups for an amino group, such as an Fmoc group, a t-butoxycarbonyl (Boc) group, a benzyloxycarbonyl (Cbz or Z) group, a p-methoxybenzyloxycarbonyl (Z(OMe) or pMZ) group, and a 2-(p-biphenyl)isopropyloxycarbonyl (Bpoc) group, and protective groups for a carboxyl group, such as a methyl ester group, an ethyl ester group, a benzyl ester group, and a t-butyl ester group. In particular, a Fmoc group and/or a methyl ester group can be used.

In the step of introducing a protective group to the glycoasparagine, the protective group may be introduced to a free carboxy group of sialic acid. Any protective group can also be applied to the protective group for a free carboxy group of sialic acid with reference to the description in relation to the protective group for the asparagine. The carboxy group may be protected by amidation with ammonia, methylamine, aromatic amine, or the like. In particular, examples thereof include protective groups for a carboxyl group, such as a methyl ester group, an ethyl ester group, a benzyl ester group, and a t-butyl ester group. In particular, a methyl ester group can be used.

In some cases, the complex-type glycoasparagine with the protective group introduced therein may be purified by, for example, but not particularly limited to, reversed-phase chromatography, hydrophilic interaction liquid chromatography, normal-phase chromatography, ion exchange chromatography, affinity chromatography, or size-exclusion chromatography.

Each chromatography can be carried out by a conventionally known method. Each chromatography may be carried out in combination with another.

The individual complex-type glycoasparagine units according to the present invention can be fractionated by further purifying the complex-type glycoasparagine. The protective group in the complex-type glycoasparagine with the protective group introduced therein can be removed for deprotection by a conventionally known method.

Examples of the support for use in reversed-phase chromatography include, but are not particularly limited to, supports in which silica is used as a base material and octadecyl, octyl, phenyl, cyanopropyl, methyl, or the like is immobilized on the surface of a packing material. In particular, ODS resin is preferred.

Examples of the support for use in hydrophilic interaction liquid chromatography include, but are not particularly limited to, supports in which silica is used as a base material and a silica gel modified with an aminopropyl group, an amide group, diol, a cyano group, a carbamoyl group, or the like, a polymer base material, or the like is immobilized on the surface of a packing material.

Examples of the mobile phase for use in chromatography include, but are not particularly limited to, water, acetonitrile, and alcohols. One solvent may be used, or a mixed solvent of two or more solvents may be used. The mobile phase may be supplemented with an acid or a base, and elution may be performed with a mobile phase under gradient conditions.

In the step of purifying the complex-type glycoasparagine separated on the basis of the number of sialic acid residues, the chromatographic purification of the complex-type glycoasparagine may be performed not only once but a plurality of times for separation and isolation on the basis of complex-type glycoasparagine units differing in glycan. The step of partially purifying the complex-type glycoasparagine may be comprised before the chromatographic purification.

### Examples

Hereinafter, the present embodiment will be described further specifically with reference to Examples and Comparative Examples. However, the present embodiment is not limited by only these Examples. Measurement methods used in the present embodiment are as follows.

### [HPLC analysis]

LC-2000 series PLUS manufactured by JASCO Corp.
Detector: UV 273 nm
Nexera Prep series manufactured by Shimadzu Corp.
Detector: UV 301 nm
Reversed-phase column: InertSustain AQ-C18 column 5 µm, 10 × 250 mm
HILIC column: InertSustain Amide column 5 µm, 10 × 250 mm

### [Mass spectrometry]

AXIMA-CFRplus manufactured by Shimadzu Corp.
Ionization: MALDI
Mode: Linear Positive
MALDI-8030 manufactured by Shimadzu Corp.
Mode: Linear Positive
[¹H-NMR]
JNE-ECA500 model FTNMR spectrometer (500 MHz) manufactured by JEOL Ltd.
Solvent: D₂O

### <Example 1>

(a) A fraction containing approximately 6.5 g of IgY was incubated at 80°C for 10 minutes, adjusted to 3.0 L with a tris-HCL/calcium chloride buffer solution (pH = 8.0), and set to a mass concentration of 0.05% by the addition of sodium azide. Further, actinase E (manufactured by Kaken Pharmaceutical Co., Ltd., 650 mg) was added thereto, and the mixture was reacted at 37°C for 48 hours.
(b) After the completion of reaction, a supernatant was recovered by centrifugation. The supernatant was purified under gradient (CH₃CN/H₂O = 90/10 → 83/17 → 75/25 --> 0/100) conditions using a column packed with cotton to recover fractions containing a glycoasparagine group.
(c) The obtained glycoasparagine fractions were applied to an anion exchange support (manufactured by JNC Corp., Cellufine A-500) to perform anion exchange chromatography. The mobile phase used was a gradient (100:0 --) 97:3 --) 80:20) of 10% acetonitrile (pH = 9.5) and 3% acetic acid:acetonitrile = 9:1 (pH = 7.3). Non-sialic acid-bound glycoasparagine was eluted when the mobile phase ratio was 100:0. Glycoasparagine bound to one sialic acid residue was eluted when the mobile phase ratio was 97:3. Glycoasparagine bound to two sialic acid residues was eluted when the mobile phase ratio was 80:20. The presence of the obtained glycoasparagine was confirmed by mass spectrometry.

### <Example 2>

(a) The glycoasparagine (neutral glycoasparagine) group having 0 sialic acid residues obtained in Example 1 was desalted using a column packed with cotton, and concentrated under reduced pressure. The obtained residue (approximately 28.0 mg) was dissolved in a water/acetone solvent (water/acetone = 3/2, 1 mL), and sodium bicarbonate (9.7 mg) was added to the solution. N-(9-Fluorenylmethoxycarbonyloxy)succinimide (Fmoc-OSu, 19.5 mg) was added thereto, and the mixture was reacted at room temperature for 2 hours. The completion of the reaction was confirmed with a TLC plate.
(b) The obtained Fmoc-protected asparagine derivative group (neutral glycoasparagine derivative group) having 0 sialic acid residues was desalted using a reversed-phase column and then subjected to reversed-phase chromatography (gradient: solution of 0.1% trifluoroacetic acid in acetonitrile/0.1% aqueous trifluoroacetic acid solution = 16/84, 3 mL/min, 140 min) using HPLC for separation on the basis of neutral glycoasparagine units differing in the number of sugars (Figure 1).
(c) A fraction eluted approximately 80 minutes later in reversed-phase chromatography was separated, concentrated under reduced pressure, and then purified by HILIC (gradient: CH₃CN/100 mM NH₄HCO₂ = 70/30 --> 65/35, 3 mL/min, 60 min) using HPLC. A fraction eluted approximately 47 minutes later was separated and desalted by gel filtration (manufactured by Cytiva/Global Life Sciences Technologies Japan K.K., Sephadex G-10) to obtain approximately 4.2 mg of a complex-type glycoasparagine derivative of 11 monosaccharides having 0 sialic acid residues and having a bisecting GlcNAc structure and a core fucose structure. ¹H-NMR data (Figure 2) and mass spectrometry results (Figure 3) of the obtained complex-type glycoasparagine derivative are shown. A complex-type glycoasparagine having a structure represented by Gal2Man3GlcNAc5Fuc (11 monosaccharides) (1) was confirmed.
(d) A fraction eluted approximately 92 minutes later in reversed-phase chromatography was separated, concentrated under reduced pressure, and then purified by HILIC (gradient: CH₃CN/100 mM NH₄HCO₂ = 71/29 → 66/34, 3 mL/min, 60 min) using HPLC. A fraction eluted approximately 38 minutes later was separated and desalted by gel filtration to obtain approximately 3.1 mg of a complex-type glycoasparagine derivative of 10 monosaccharides having 0 sialic acid residues and having a bisecting GlcNAc structure and a core fucose structure. ¹H-NMR data (Figure 4) and mass spectrometry results (Figure 5) of the obtained complex-type glycoasparagine derivative are shown. A complex-type glycoasparagine having a structure represented by GalMan3GlcNAc5Fuc (10 monosaccharides) (1) was confirmed.
(e) A fraction eluted approximately 102 minutes later in reversed-phase chromatography was separated, concentrated under reduced pressure, and then purified by HILIC (gradient: CH₃CN/100 mM NH₄HCO₂ = 71/29 --> 66/34, 3 mL/min, 60 min) using HPLC. A fraction eluted approximately 45 minutes later was separated and desalted by gel filtration to obtain a complex-type glycoasparagine derivative of 10 monosaccharides having 0 sialic acid residues and having a bisecting GlcNAc structure and a core fucose structure. Mass spectrometry results (Figure 6) of the obtained complex-type glycoasparagine derivative are shown. A complex-type glycoasparagine having a structure represented by GalMan3GlcNAc5Fuc (10 pentasaccharide) (2) was confirmed.
(f) A fraction eluted approximately 118 minutes later in reversed-phase chromatography was separated, concentrated under reduced pressure, and then purified by HILIC (gradient: CH₃CN/100 mM NH₄HCO₂ = 72/28 --> 67/33, 3 mL/min, 60 min) using HPLC. A fraction eluted approximately 38 minutes later was separated and desalted by gel filtration to obtain a complex-type glycoasparagine derivative of 9 monosaccharides having 0 sialic acid residues and having a bisecting GlcNAc structure and a core fucose structure. Mass spectrometry results (Figure 7) of the obtained complex-type glycoasparagine derivative are shown. A complex-type glycoasparagine having a structure represented by Man3GlcNAc5Fuc (9 monosaccharides) was confirmed.

### <Example 3>

(a) The glycoasparagine group having one sialic acid residue obtained in Example 1 was desalted using a column packed with cotton, and concentrated under reduced pressure. The obtained residue (approximately 18.2 mg) was dissolved in a water/acetone solvent (water/acetone = 3/2, 1 mL), and sodium bicarbonate (5.2 mg) was added to the solution. N-(9-Fluorenylmethoxycarbonyloxy)succinimide (Fmoc-OSu, 10.4 mg) was added thereto, and the mixture was reacted at room temperature for 2 hours. The completion of the reaction was confirmed with a TLC plate.
(b) The obtained Fmoc-protected asparagine derivative group (acidic glycoasparagine derivative group) having one sialic acid residue was desalted using a reversed-phase column and then subjected to reversed-phase chromatography (gradient: solution of 0.1% trifluoroacetic acid in acetonitrile/0.1% aqueous trifluoroacetic acid solution = 17/83, 3 mL/min, 60 min) using HPLC to recover a fraction containing an asparagine derivative having one sialic acid residue eluted approximately 40 minutes later.
(c) The fraction eluted approximately 40 minutes later in reversed-phase chromatography was separated, concentrated under reduced pressure, and then purified by HILIC (gradient: CH₃CN/100 mM NH₄HCO₂ = 67/33 --> 62/38, 3 mL/min, 45 min) using HPLC. A fraction eluted approximately 35 minutes later was separated and subjected again to reversed-phase chromatography with the mobile phase changed (gradient: MeOH/50 mM NH₄HCO₂ = 35/65 --> 40/60, 4 mL/min, 60 min) to separate a fraction eluted approximately 30 minutes later. The fraction was desalted by gel filtration to obtain approximately 2.8 mg of a complex-type glycoasparagine derivative of 12 monosaccharides having one sialic acid residue and having a bisecting GlcNAc structure and a core fucose structure. ¹H-NMR data (Figure 8) of the obtained complex-type glycoasparagine derivative is shown. A complex-type glycoasparagine having a structure represented by Neu5AcGal2Man3GlcNAc5Fuc (12 monosaccharides) (1) was confirmed.
(d) To an aliquot of the obtained complex-type glycoasparagine derivative, MeOH (20 µL), 1-(3-dimethylaminopropyl)-3-ethylcarbodiimide hydrochloride (250 mM), and 1-hydroxybenzotriazole monohydrate (250 mM) were added, and the mixture was reacted at 37°C for 1 hour. Mass spectrometry results (Figure 9) of the obtained methylated derivative of the complex-type glycoasparagine are shown. A complex-type glycoasparagine having a structure represented by Neu5AcGal2Man3GlcNAc5Fuc (12 monosaccharides) (1) was confirmed.

### <Example 4>

(a) The glycoasparagine group having two sialic acid residues obtained in Example 1 was desalted using a column packed with cotton, and concentrated under reduced pressure. To an aliquot of the obtained residue (approximately 1.2 mg), MeOH (20 µL), 1-(3-dimethylaminopropyl)-3-ethylcarbodiimide hydrochloride (250 mM), and 1-hydroxybenzotriazole monohydrate (250 mM) were added, and the mixture was reacted at 37°C for 1 hour. Mass spectrometry results (Figure 10) of the obtained methylated derivative of the complex-type glycoasparagine are shown. A complex-type glycoasparagine having a structure represented by Neu5Ac2Gal2Man3GlcNAc5Fuc (13 monosaccharides) was confirmed.

### <Example 5>

(a) Approximately 100 g of IgY was treated in the same manner as in Examples 1 and 2 to obtain 3.0 mg of GalMan3GlcNAc5Fuc (10 monosaccharides) (2) described in Example 2(e). ¹H-NMR data (Figure 11) of the obtained complex-type glycan is shown.

### <Example 6>

(a) Approximately 100 g of IgY was treated in the same manner as in Examples 1 and 2 to obtain 9.3 mg of Man3GlcNAc5Fuc (9 monosaccharides) described in Example 2(f). ¹H-NMR data (Figure 12) of the obtained complex-type glycan is shown.

### <Example 7>

(a) Approximately 100 g of IgY was treated in the same manner as in Examples 1 and 4 to obtain 174.1 mg of a glycoasparagine group having two sialic acid residues. The obtained glycoasparagine group having two sialic acid residues was dissolved in a water/acetone solvent (water/acetone = 3/2, 18 mL), and sodium bicarbonate (117.01 mg) was added to the solution. N-(9-Fluorenylmethoxycarbonyloxy)succinimide (Fmoc-OSu, 191.1 mg) was added thereto, and the mixture was reacted at room temperature for 4 hours. The completion of the reaction was confirmed with a TLC plate.
(b) The obtained Fmoc-protected asparagine derivative group having two sialic acid residues was desalted using a reversed-phase column and then purified by HILIC (gradient: CH₃CN/50 mM NH₄HCO₂ = 72/28 --> 69/31, 3 mL/min, 85 min) using HPLC to recover a fraction containing an asparagine derivative having two sialic acid residues eluted approximately 68 minutes later. The obtained fraction was concentrated under reduced pressure and then subjected to reversed-phase chromatography (gradient: acetonitrile/50 mM aqueous ammonium formate solution = 12/88 --> 17/83, 5 mL/min, 60 min) using HPLC to recover a fraction containing an asparagine derivative having two sialic acid residues eluted approximately 47 minutes later. The fraction was desalted by gel filtration to obtain approximately 10.0 mg of a complex-type glycoasparagine derivative of 13 monosaccharides having two sialic acid residues and having a bisecting GlcNAc structure and a core fucose structure. ¹H-NMR data (Figure 13) of the obtained complex-type glycoasparagine derivative is shown. A complex-type glycoasparagine having a structure represented by Neu5Ac2Gal2Man3GlcNAc5Fuc (13 monosaccharides) was confirmed.
(c) To an aliquot of the obtained complex-type glycoasparagine derivative, MeOH (20 µL), 1-(3-dimethylaminopropyl)-3-ethylcarbodiimide hydrochloride (250 mM), and 1-hydroxybenzotriazole monohydrate (250 mM) were added, and the mixture was reacted at 37°C for 1 hour. Mass spectrometry results (Figure 14) of the obtained methylated derivative (Fmoc-protected form) of the complex-type glycoasparagine are shown. A complex-type glycoasparagine having a structure represented by Neu5Ac2Gal2Man3GlcNAc5Fuc (13 monosaccharides) was confirmed.

## Claims

1. A complex-type glycoasparagine derived from an avian antibody,
the complex-type glycoasparagine having a bisecting GlcNAc structure and a core fucose structure, wherein an Asn residue is optionally protected.

2. The complex-type glycoasparagine according to claim 1, wherein a glycan of the complex-type glycoasparagine has any of 9 to 13 monosaccharides, wherein the Asn residue is optionally protected.

3. The complex-type glycoasparagine according to claim 1 or 2, wherein the complex-type glycoasparagine has the following structure: 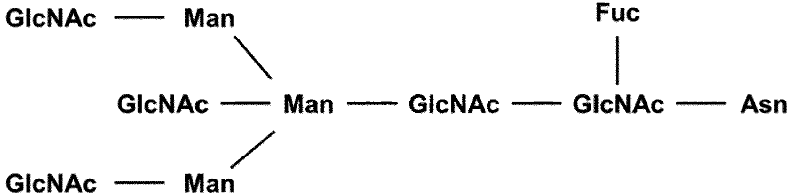 wherein the Asn residue is optionally protected.

4. The complex-type glycoasparagine according to claim 3, wherein Gal or Neu5Ac-Gal is bound to one or both of GlcNAc in the GlcNAc-Man-Man moieties, wherein the Asn residue is optionally protected.

5. A method for producing a complex-type glycoasparagine, comprising the steps of:
providing a mixture containing a complex-type glycoasparagine derived from an avian antibody; and
purifying the complex-type glycoasparagine by allowing the mixture to pass through an anion exchange resin,
wherein in the purification step, the complex-type glycoasparagine is separated on the basis of the number of sialic acid residues.

6. The method according to claim 5, wherein the mixture provision step comprises the steps of:
denaturing the avian antibody; and
degrading the denatured avian antibody with a proteolytic enzyme.

7. The method according to claim 6, wherein, in the avian antibody denaturation step, the avian antibody is denatured with heat or an organic solvent.
